# EUROPEAN PATENT SPECIFICATION

(11) **EP 2 252 376 B1**
(45) Date of publication and mention of the grant of the patent: **19.11.2014**
(21) Application number: 08725109.6
(22) Date of filing: 01.02.2008
(51) Int. Cl.: A62B 7/08, G05D 7/00

(54) **UV-PCO AIR PURIFIER WITH CONTROL SYSTEM**
UV-PCO-LUFTREINIGER MIT STEUERSYSTEM
PURIFICATEUR D'AIR UV-PCO AVEC SYSTÈME DE COMMANDE

(43) Date of publication of application: 24.11.2010
(73) Proprietor: Carrier Corporation, Farmington, CT 06034-4015 (US)
(72) Inventor: LEMCOFF, Norberto, O., Farmington, CT 06034-4015 (US); HAY, Stephen, O., Tolland, CT 06084 (US); OBEE, Timothy, N., Farmington, CT 06034-4015 (US); THIBAUD-ERKEY, Catherine, Farmington, CT 06034-4015 (US); BRANDES, Leland, G., South Windsor, Connecticut 06074 (US); BRANDES, Susan, D. (deceased), . (US)
(74) Representative: Leckey, David Herbert
(86) International application number: PCT/US2008/001420
(87) International publication number: WO 2009/096924

(56) References cited:
- EP-A2- 0 826 531
- EP-A2- 1 640 671
- WO-A1-2009/002295
- WO-A2-2006/065491
- WO-A2-2007/143041
- US-A- 5 948 355
- US-A- 6 063 343
- US-A1- 2006 188 387
- US-A1- 2007 193 875

## Description

### BACKGROUND

This invention relates generally to the use of ultraviolet photocatalytic oxidation (UV-PCO) technology for decontamination of air in air purification systems. More specifically, the present invention relates to a control system and method for coordinating operation of components of the air purifier system when the system is first turned on after a period of inactivity.

Some buildings utilize air purification systems to remove airborne substances such as benzene, formaldehyde, and other contaminants from the air supply. Some of these purification systems include photocatalytic reactors that utilize a substrate or cartridge containing a photocatalyst. When placed under an appropriate light source, typically a UV light source, the photocatalyst interacts with oxygen and airborne water molecules to form active oxidation species such as hydroxyl radicals. The hydroxyl radicals then attack the contaminants and initiate an oxidation reaction that converts the contaminants into less harmful compounds, such as water and carbon dioxide. It is further believed that the combination of oxygen, water vapor, suitably energetic photons and a photocatalyst also generates an active oxygen agent like hydrogen peroxide. [W. Kubo and T. Tatsuma, Analytical Sciences, Vol. 20, 591-93 (2004)].

UV-PCO air purification systems are attractive because they convert volatile organic compounds (VOCs) to harmless compounds. The most common types of VOCs, which are pure hydrocarbons, are converted to water and carbon dioxide by the UV-PCO process. The typical operation of a UV-PCO involves periodic rather than continuous operation of the air purifier. The air purifier may be turned on by a timer, or by a control signal from an HVAC system. Typically, the photocatalytic reactor begins cleansing a flow of contaminated air when the UV-PCO air purifier is turned on.

Photocatalytic air treatment systems having the features of the preamble of claim 1 are disclosed in WO 2006/065491, WO 2007/143041, EP-A-1640671 and EP-A-826531. A further purification system is disclosed in WO 2009/002295.

### SUMMARY

The present invention is based upon the recognition that a UV-PCO air purifier may have been turned off for a considerable amount of time before it is turned on. During this off time period, considerable adsorption of volatile organic compounds may occur either on the photocatalyst surface of the reactor, or on an upstream filter. The concentration of volatile organic compounds can be excessively high. If this concentration of volatile organic compounds is present when the UV source of the photocatalytic reactor is turned on, the reaction occurring on the catalyst surface can lead to either incomplete oxidation or to generation of high molecular weight compounds that strongly adsorb on the photocatalyst surface and prevent other species from reaching the photocatalyst. As a result, the photocatalyst can suffer loses in activity, and the air purifier will suffer from reduced effectiveness.

From a first aspect, the invention provides an air purification system as set forth in claim 1. From a second aspect, the invention provides a method as set forth in claim 4.

### BRIEF DESCRIPTION OF THE DRAWINGS

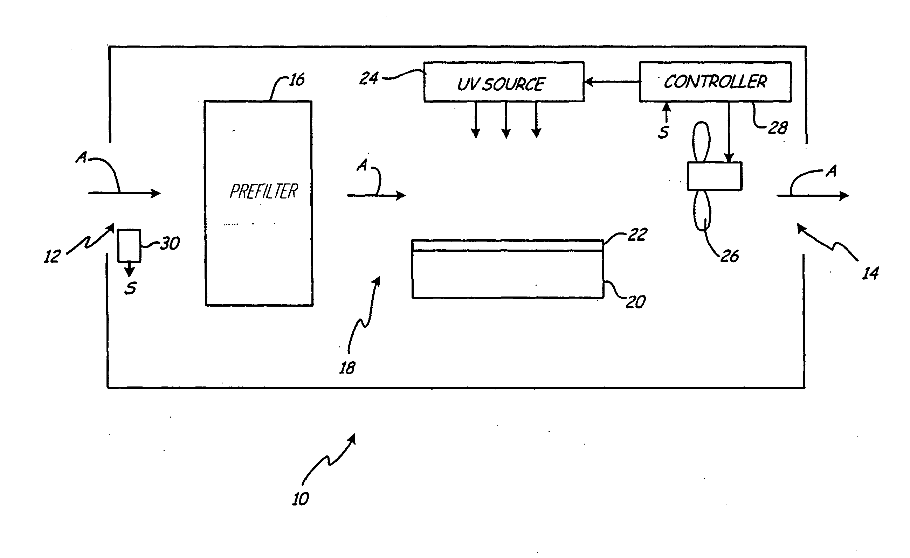

The sole FIGURE is a block diagram schematically illustrating a photocatalytic air purification device.

### DETAILED DESCRIPTION

The FIGURE is a schematic diagram of air purifier system 10, which uses ultraviolet photocatalytic oxidation (UV-PCO) to remove contaminants from air. Air purifier system includes inlet 12, outlet 14, prefilter 16, photocatalytic reactor 18 (which includes substrate 20, photocatalytic coating 22, and UV source 24), fan 26 and controller 28.

Airstream A passes through prefilter 16 and then through photocatalytic reactor 18 and fan 26 to outlet 14. Prefilter 16 removes dust and particles from airstream A before they reach photocatalytic reactor 18. Prefilter 16 may contain a carbon filter to remove VOCs such as volatile silicon-containing compounds (VSCCs) from airstream A.

As airstream A passes through photocatalytic reactor 18, it comes in contact with photocatalytic coating 22. In the FIGURE, substrate 20 is illustrated schematically as a flat plate. In practice, substrate 20 can take a number of different forms, which may be configured to maximize surface area on which photocatalytic coating 22 is located (and thus maximize the surface area in which contact between photocatalytic coating 22 and airstream A can take place). One example is a honeycomb structure on which photocatalytic coating 22 is deposited and through which airstream A passes.

Ultraviolet radiation from UV source 24 is directed for and is absorbed by photocatalyst coating 22. The UV radiation causes photocatalyst coating 22 to interact with airborne water molecules to produce reactive species such as hydroxyl radicals, hydrogen peroxide, hydrogen peroxide radicals, and super oxide ions. These reactive species interact with VOCs in airstream A to transform VOCs into harmless byproducts such as carbon dioxide and water. Therefore, airstream A contains less contaminants as it exits system 10 through outlet 14 than it contained when it entered system 10 through inlet 12.

Controller 28 coordinates the operation of a regeneration mode and photocatalytic reactor 18. System 10 typically operates intermittently or periodically, rather than on a continuous basis. Controller 28, which may be, for example, a microprocessor based controller may receive commands from an HVAC system to initiate operation of air purifier system 10. Alternatively, controller 28 may be programmed to initiate and terminate operation of system 10 based upon a stored operating schedule or upon sensed parameters.

When system 10 is turned on, either by an external command received by controller 28 or as a result of a determination made by controller 28, the regeneration mode is operated for a period of time before air is purified or cleaned. Different regeneration modes can be used according to the type of contaminant present. For example, compounds which contain only hydrogen, carbon, and oxygen atoms usually only cause reversible damage. Example compounds include hydrocarbons such as toluene, xylene and ethylbenzene. On the other hand, volatile or semi-volatile organic compounds containing heteroatoms such as silicon, nitrogen, phosphorus and/or sulfur can lead to irreversible deactivation.

In an embodiment in accordance with the claimed invention, the regeneration mode uses fan 26 to regenerate a surface in system 10. Fan 26 causes airflow through system 10 from inlet 12 to outlet 14. This airflow allows the concentration of VOCs that may have been adsorbed during the off period of system 10 to be moved through system 10 before UV source 24 is turned on and before photocatalyst coating 22 begins conversion of VOCs into harmless products.

The concentration of VOCs that may have accumulated on pre-filter 16 or on photocatalyst coating 22 during a period of inactivity of system 10 could adversely affect the operation of system 10 if UV source 24 is turned on immediately when system 10 is turned on. A high concentration of VOCs could result in incomplete oxidation or generation of high molecular weight compound that cause photocatalyst 22 to have reduced photocatalytic activity.

The period of time that fan 26 operates before UV source 24 is turned on can be a programmed time period within controller 28. A typical amount of time may be, for example, between about 5 minutes and about 10 minutes.

Controller 28 may include a real-time clock or other timer circuitry to determine how long system 10 was inactive before receiving a command to turn on. If the period of inactivity is relatively short, the time delay between operation of fan 26 and UV source 24 may be reduced, or may be eliminated in some circumstances. The amount of time required for fan 26 to move air through pre-filter 16 and photocatalytic reactor 18 may be controlled, therefore, as a function of the inactive period during which VOCs have been allowed to accumulate within system 10.

By not turning on UV source 24 when a high concentration of VOCs is adsorbed on photocatalyst coating 22, the deactivation of photocatalyst coating 22 is significantly reduced. The time delay between operation of fan 26 and UV source 24 does not significantly affect the overall operation of system 10 in its ability to remove contaminants from ambient air.

In an arrangement falling outside the scope of the claimed invention, the regeneration mode uses UV source 24 to regenerate photocatalyst coating 22 when compounds that cause reversible damage to photocatalyst coating 22 are present. For example, when photocatalyst coating 22 has been exposed to high levels of hydrocarbons (HCs), the hydrocarbons occupy some or all of the catalyst's active sites and are not efficiently and quickly removed from the surface just by purging it with clean air. UV light and a minimum flow of clean air removes these contaminates from photocatalyst coating 22.

Examples of high levels of hydrocarbons include when the total hydrocarbon concentration is 100 parts per billion or higher or when a specific hydrocarbon concentration (such as the toluene concentration) is 50 parts per billion or higher. Example hydrocarbons include hydrocarbons that are capable of chemisorbing onto a surface of system 10, such as toluene, xylene and ethylbenzene.

The minimum flow of clean air is the incidental or natural air flow through system 10 that is caused by the local heating of the air by UV source 24, and the clean air may be from the same source as the air which flows through system 10 during the operation of system 10. After flowing through system 10, the clean air may be directed to the building air supply or alternatively may be exhausted outside through vents.

The period of time that UV source 24 operates with a minimum flow of clean air before contaminated air is admitted into reactor 18 can be a programmed time period within controller 28. A typical amount of time may be, for example, between about 2 to about 8 hours.

Air purification system 10 may also include hydrocarbon (HC) measurement device 30, which is located upstream of photocatalytic reactor 18, such as at inlet 12. HC measurement device 30 measures the hydrocarbon concentration of airstream A. HC measurement device 30 may measure the total hydrocarbon concentration of airstream A, a specific hydrocarbon concentration, such as the toluene concentration, of airstream A, or any combination thereof. HC measuring device 30 may use gravimetric, thermal, resistive, electronic, magnetic, photolytic, optical or related sensing strategies, or any combination thereof, as the means of measuring the hydrocarbon concentration. HC measurement device 30 may send signal S, which represents the measured hydrocarbon concentration, to controller 28 to determine the appropriate operation procedure for system 10 after a prolonged period of inactivity. For example, the controller may only initiate a regeneration mode when a total hydrocarbon concentration of 100 parts per billion or higher is measured by HC measurement device 30, or when a specific hydrocarbon concentration, such as a toluene concentration, of 50 parts per billion or higher is measured by HC measurement device 30.

The regeneration mode used is based upon the environment in which the system is installed. Some environments may require using a plurality of regeneration modes. For example, in one system a regeneration mode performed by running a fan to remove VOCs is performed everyday while a regeneration mode performed by using a UV light source to remove hydrocarbons is performed on weekends. In another example, two different regeneration modes are performed in series in a system, such as regenerating a surface by removing VOCs followed by regenerating the same or different surface in the system by removing hydrocarbons.

## Claims

1. An air purification system (10) comprising:
an inlet (12);
an outlet (14);
a photocatalytic reactor (18) for removing volatile organic compounds (VOCs) from air;
a fan (26); and
a UV source (24); **characterised by** further comprising
a controller for coordinating operation of the system so that upon the system beginning operation, the controller (28) causes the system (10) to operate in a regeneration mode for a time period to regenerate the photocatalytic reactor by removing contaminants adsorbed during non-operation of the system (10) by operating the fan (26) to cause air to enter the system (10) through the inlet (12), to move through the photocatalytic reactor (18), and to exit the system (10) through the outlet (14) before the controller (28) initiates a normal operation mode by activating the photocatalytic reactor (18) to cleanse the air by activating the UV source (24) and the fan (26); and
wherein a period of time that the fan operates before the UV source (24) is turned on is a programmed time period within the controller (28), or
is a function of a time duration of inactivity of the photocatalytic reactor prior to beginning operation of the fan (26).

2. The air purification system of claim 1, wherein the programmed time period is 5 minutes to 10 minutes.

3. The air purification system of any preceding claim and further comprising:
a prefilter (16) for removing particulates from the air, the prefilter at a location upstream of the photocatalytic reactor.

4. A method of removing volatile organic compounds (VOCs) from air, the method comprising:
regenerating a surface of an air purification system (10) for an initial time period to dissipate contaminants accumulated during non-operation of a photocatalytic reactor (18) by directing air through the photocatalytic reactor (18) for an initial time period to dissipate VOCs using a fan (26); and
activating the photocatalytic reactor (18) by activating a UV source (24) and the fan (26) following the initial time period;
wherein a period of time that the fan operates before the UV source (24) is turned on is a programmed time period within the controller (28), or
is a function of a time duration of inactivity of the photocatalytic reactor prior to beginning operation of the fan (26).

5. The method of claim 4, wherein the programmed time period is 5 to 10 minutes.

## Patentansprüche

1. Luftreinigungssystem (10), umfassend:
einen Einlass (12);
einen Auslass (14);
einen fotokatalytischen Reaktor (18) zum Entfernen flüchtiger organischer Verbindungen (VOCs) aus Luft;
ein Gebläse (26); und
eine UV-Quelle (24); **dadurch gekennzeichnet, dass** es ferner Folgendes umfasst:
eine Steuerung zum Koordinieren des Betriebs des Systems derart, dass bei Beginn des Systembetriebs die Steuerung (28) das System (10) veranlasst, für einen Zeitraum in einem Regenerationsmodus zu arbeiten, um den fotokatalytischen Reaktor zu regenerieren, indem Verunreinigungen, die während des Nicht-Betriebs des Systems (10) adsorbiert wurden, entfernt werden, indem das Gebläse (26) betrieben wird, um zu bewirken, dass Luft durch den Einlass (12) in das System (10) gelangt und sich durch den fotokatalytischen Reaktor (18) bewegt und das System (10) durch den Auslass (14) zu verlassen, bevor die Steuerung (28) einen Normalbetriebsmodus einleitet, indem sie den fotokatalytischen Reaktor (18) aktiviert, um die Luft durch Aktivieren der UV-Quelle (24) und des Gebläses (26) zu reinigen; und
wobei ein Zeitraum, für den das Gebläse arbeitet, bevor die UV-Quelle (24) eingeschaltet wird, ein programmierter Zeitraum innerhalb der Steuerung (28) ist, oder
eine Funktion eines Zeitraums der Inaktivität des fotokatalytischen Reaktors vor dem Betriebsbeginn des Gebläses (26) ist.

2. Luftreinigungssystem nach Anspruch 1, wobei der programmierte Zeitraum 5 Minuten bis 10 Minuten beträgt.

3. Luftreinigungssystem nach einem der vorangehenden Ansprüche und ferner umfassend:
einen Vorfilter (16) zum Entfernen von Partikeln aus der Luft, wobei der Vorfilter an einer Position ist, die dem fotokatalytischen Reaktor vorgelagert ist.

4. Verfahren zum Entfernen flüchtiger organischer Verbindungen (VOCs) aus Luft, wobei das Verfahren Folgendes umfasst:
Regenerieren einer Oberfläche eines Luftreinigungssystems (10) für einen Anfangszeitraum, um Verunreinigungen, die sich während des Nicht-Betriebs eines fotokatalytischen Reaktors (18) angesammelt haben, zu zerstreuen, indem für den Anfangszeitraum Luft durch den fotokatalytischen Reaktor (18) gelenkt wird, um VOCs mithilfe eines Gebläses (26) zu zerstreuen; und
Aktivieren des fotokatalytischen Reaktors (18) durch Aktivieren einer UV-Quelle (24) und des Gebläses (26) nach dem Anfangszeitraum;
wobei ein Zeitraum, für den das Gebläse arbeitet, bevor die UV-Quelle (24) eingeschaltet wird, ein programmierter Zeitraum innerhalb der Steuerung (28) ist, oder
eine Funktion eines Zeitraums der Inaktivität des fotokatalytischen Reaktors vor dem Betriebsbeginn des Gebläses (26) ist.

5. Verfahren nach Anspruch 4, wobei der programmierte Zeitraum 5 Minuten bis 10 Minuten beträgt.

## Revendications

1. Système de purification d'air (10) comprenant :
une entrée (12) ;
une sortie (14) ;
un réacteur photocatalytique (18) permettant d'éliminer des composés organiques volatils (COV) de l'air ;
un ventilateur (26) ; et
une source d'UV (24) ; **caractérisé en ce qu'**il contient en outre
un contrôleur servant à coordonner le fonctionnement du système de sorte que lorsque le système commence à fonctionner, le contrôleur (28) amène le système (10) à opérer selon un mode de régénération pendant une durée pour régénérer le réacteur photocatalytique en éliminant les contaminants adsorbés pendant le non-fonctionnement du système (10) par actionnement du ventilateur (26), ce qui amène l'air à pénétrer dans le système (10) à travers l'entrée (12), à traverser le réacteur photocatalytique (18) et à sortir du système (10) à travers la sortie (14) avant que le contrôleur (28) ne lance un mode normal de fonctionnement par activation du réacteur photocatalytique (18) servant à nettoyer l'air par activation de la source d'UV (24) et le ventilateur (26) ; et
dans lequel une durée où le ventilateur fonctionne avant que la source d'UV (24) ne soit mise en marche est une durée programmée au sein du contrôleur (28), ou
est une fonction d'une durée d'inactivité du réacteur photocatalytique avant de commencer le fonctionnement du ventilateur (26).

2. Système de purification d'air selon la revendication 1, dans lequel la durée programmée est de 5 à 10 min.

3. Système de purification d'air selon l'une quelconque des revendications précédentes, comprenant en outre : un préfiltre (16) servant à éliminer des particules de l'air, le préfiltre se trouvant en un emplacement situé en amont du réacteur photocatalytique.

4. Procédé d'élimination de composés organiques volatils (COV) de l'air, ce procédé comprenant :
la régénération d'une surface d'un système de purification d'air (10) pendant une durée initiale, afin de dissiper les contaminants accumulés pendant le non-fonctionnement d'un réacteur photocatalytique (18) par acheminement d'air à travers le réacteur photocatalytique (18) pendant une durée initiale, afin de dissiper les COV à l'aide d'un ventilateur (26) ; et
l'activation du réacteur photocatalytique (18) par activation d'une source d'UV (24) et du ventilateur (26) après la durée initiale ;
une durée où le ventilateur fonctionne avant que la source d'UV (24) ne soit mise en marche étant programmée dans le contrôleur (28), ou
est une fonction d'une durée d'inactivité du réacteur photocatalytique avant de commencer le fonctionnement du ventilateur (26).

5. Procédé selon la revendication 4, dans lequel la durée programmée est de 5 à 10 min.
